# FASCICULE DE BREVET EUROPEEN

(11) **EP 3 641 669 B1**
(45) Date de publication et mention de la délivrance du brevet: **26.05.2021**
(21) Numéro de dépôt: 18745998.7
(22) Date de dépôt: 22.06.2018
(51) Int. Cl.: A61B 17/17

(54) **SYSTÈME D'INSERTION ET DE RETRAIT D'UNE BROCHE REPÈRE DANS UN OS**
SYSTEM ZUM EINSETZEN UND ENTFERNEN EINES REFERENZSTIFTES IN EINEN / AUS EINEM KNOCHEN
SYSTEM FOR INSERTING AND REMOVING A REFERENCE PIN INTO/OUT OF A BONE

(30) Priorité: 23.06.2017 FR 1770666; 23.06.2017 US 201715631081
(43) Date de publication de la demande: 29.04.2020
(73) Titulaire: Stylitech, 34090 Montpellier (FR)
(72) Inventeur: MARNAY, Thierry, 34090 Montpellier (FR)
(74) Mandataire: Touroude, Magali Linda
(86) Numéro de dépôt international: PCT/FR2018/000178
(87) Numéro de publication internationale: WO 2018/234644

(56) Documents cités:
- US-A- 5 196 015
- US-A1- 2007 270 896
- US-A1- 2010 168 751

## Description

La présente invention se rapporte à un système d'insertion et de retrait d'une broche repère dans un os, à une broche repère, à un porte-broche d'insertion et à un porte-broche de retrait.

Plus particulièrement, l'invention concerne un système utilisé pour la chirurgie vertébrale, en particulier pour la mise en place d'implants de type vis par exemple, pour la stabilisation des fractures des tumeurs, l'immobilisation du rachis en cas d'arthrodèse (greffe intervertébrale afin de souder deux vertèbres ou plus) dans la pathologie dégénérative, et pour la correction des déformations de la colonne vertébrale (scolioses et cyphoses).

Depuis les années 60, la chirurgie vertébrale a vu le développement d'une ostéosynthèse performante permettant de réduire et fixer les fractures, d'immobiliser les arthrodèses et surtout de corriger les déformations (scolioses et cyphoses). Après l'utilisation de crochets, l'usage des vis pédiculaires s'est répandu de manière universelle.

Les vis pédiculaires sont insérées dans les vertèbres à des endroits très spécifiques, les pédicules, pour permettre de corriger les déformations sans risque d'endommager la colonne vertébrale ou son contenu, la moelle épinière. Pour cela, il est nécessaire de procéder à un repérage précis avant insertion. US 2007/270896 divulgue un système comprenant un outil d'accès au pédicule permettant le positionnement d'une vis pédiculaire.

Si les repères anatomiques ont été étudiés depuis longtemps afin de rendre cette mise en place fiable de manière répétitive, il n'en demeure pas moins que les conditions de mise en place de ces vis pédiculaires sur les vertèbres peuvent se révéler délicates en particulier dans les déformations de la colonne vertébrale associées à un trouble rotatoire. Enfin, en zone de rachis thoracique, la « visée pédiculaire » réunit tous les éléments de difficulté : repères anatomiques plus délicats voire impossibles en cas de rotation des corps vertébraux, pédicules de petite taille et risque de lésion médullaire majeure en cas d'effraction corticale ou de fausse route.

De nombreux outils d'assistance ont été développés afin d'optimiser la sécurité et l'efficience des vis pédiculaires mais ils sont insuffisants.

Ainsi, la chirurgie classique de redressement de la colonne vertébrale se déroule intégralement dans une salle d'opération spécifique, équipée d'un système d'imagerie, généralement un amplificateur de brillance (fluoroscopie).

Dans un premier temps, le chirurgien ouvre le patient et prend ses repères anatomiques en localisant l'entrée des pédicules.

Puis à l'aide des radios faites au moment du diagnostic, il évalue la position des pédicules, leur orientation et l'état des structures internes afin d'orienter sa balistique, c'est-à-dire l'angle selon lequel il va percer les pédicules.

Le chirurgien procède ensuite au percement dans les positions (place, angle, profondeur) qu'il a déterminées. Ceci est fait à l'aide d'un «trocart» constitué d'une tige munie d'une pointe trocart capable de percer l'os.

Une fois l'os percé, il contrôle le positionnement du trocart avec l'amplificateur de brillance avant de le retirer et de le remplacer par une vis pédiculaire.

Le taraudage de l'os par la vis est fait « à main levée », c'est-à-dire sans guidage et sans visibilité de la structure interne de l'os, ce qui peut entrainer des dommages sur la structure osseuse, en particulier si elle est fragile. En outre, la vis peut ne pas suivre parfaitement le trou réalisé précédemment avec le trocart.

Une fois les vis pédiculaires fixées, le chirurgien positionne les tiges de courbure et les tiges de renfort, puis les fixe en position.

Toute l'opération nécessite une ouverture importante du patient afin de laisser suffisamment de place au chirurgien pour mettre en œuvre le vissage des vis pédiculaires, le cintrage des tiges de courbure, et le positionnement définitif des tiges de courbure et des tiges de renfort.

Cependant, en cas d'anatomie complexe ou de forte déformation de la colonne vertébrale (par exemple en cas de rotation de la zone thoracique), l'amplificateur de brillance est insuffisant pour obtenir un contrôle précis des percements et entraine une importante irradiation.

Il est alors nécessaire d'utiliser d'autres types d'outils d'assistance.

On connait, par exemple, les systèmes informatisés dits « de navigation », avec recalibrage d'images de scanner préalables grâce à l'amplificateur de brillance. Cependant, ils sont difficiles à fiabiliser et lourds à mettre en oeuvre. En effet, les images de scanner sont effectuées patient sur le dos alors que l'intervention doit se faire patient sur le ventre.

En outre, les points anatomiques relevés pour recaler les images imposent de longues séquences de relevés topographiques sans obtenir une précision absolue puisque la colonne est un système articulé et mobile entre chacun de ses éléments, et donc susceptible de modifier sa position ne serait-ce que par les mouvements respiratoires.

On connait également le scanner type « O-Arm », dont la précision des enregistrements permet de palier certains de ces inconvénients mais son encombrement, le temps de mise en place, la difficulté d'associer le champ opératoire, la prise d'images scanner et les gestes chirurgicaux rendent leur utilisation très délicate et pesante. De plus, l'encombrement d'un tel système est préjudiciable à la réalisation de l'acte chirurgical. Son utilisation impose la protection du personnel (chirurgien, anesthésiste, personnel infirmier et aides opératoires) en cours d'intervention ce qui complexifie l'usage répétitif de ces appareils lorsqu'une instrumentation nécessite d'être pratiquée sur toute la hauteur de la colonne vertébrale. Se pose enfin les problèmes d'asepsie de ce type de repérage sur un patient en cours de procédure chirurgicale ouverte.

Il a également été proposé d'utiliser un robot pour effectuer le vissage des vis pédiculaires.

Cependant, ce type de robots nécessite là encore une précision millimétrique et au degré près, avec des recalibrages itératifs au fur et à mesure que l'on s'éloigne du bassin. La rotation des corps vertébraux est également un écueil important pour la fiabilité du système, surtout en zone thoracique. La nécessité d'un scanner opératoire de type «O-Arm» associé au robot rend l'encombrement général préjudiciable à l'acte chirurgical lui-même. Enfin, l'obligation d'une table opératoire en carbone vient renchérir un coût d'équipement déjà très élevé.

L'objectif de la présente invention est de proposer un dispositif économique et très précis, permettant la mise en oeuvre d'une procédure technique économique, originale, sécurisée et réduisant l'exposition chirurgicale.

L'invention permet une sécurité nettement améliorée (amélioration de l'asepsie générale de l'opération, réduction du saignement, du risque neurologique et des complications opératoires), une limitation de l'exposition aux rayonnements, un positionnement optimal des implants y compris dans des zones jusque-là non exploitées, en combinant une chirurgie mini-invasive et la radiologie interventionnelle sous scanner.

Ainsi, l'invention propose de scinder l'opération en deux phases distinctes : une en salle de radiologie, l'autre en salle opératoire.

Pour cela, l'invention propose une broche dite « repère » à usage temporaire, ainsi qu'un système d'insertion et de retrait de ladite broche repère, afin de permettre le passage entre la phase en salle de radiologie et la phase opératoire en salle d'opération tout en améliorant la précision du positionnement des vis pédiculaires grâce à une transmission optimale de l'information entre le radiologue et le chirurgien.

À cette fin, l'invention a pour objet un système d'insertion et de retrait d'une broche dite « repère » pour le positionnement d'une vis pédiculaire dans un os, le système comprenant :
- Une broche repère comprenant :
   o Un corps de broche ;
   ∘ Une portion distale conformée pour percer un os ; et
   o Une portion proximale munie :
      ▪ D'un premier moyen de fixation pour la fixation, en utilisation, à un porte-broche d'insertion ; et
      ▪ D'un second moyen de fixation pour la fixation, en utilisation, à un porte-broche de retrait ;
      les deux moyens de fixation étant différents ;
- un porte-broche d'insertion muni d'un moyen de fixation complémentaire du premier moyen de fixation de la broche repère ;
- un porte-broche de retrait muni d'un moyen de fixation complémentaire du second moyen de fixation de la broche repère ; et
- une canule de guidage destinée à recevoir en coulissement libre la broche repère, le porte-broche d'insertion et le porte-broche de retrait.

L'invention a également pour objet une broche dite «repère" pour le positionnement d'une vis pédiculaire dans un os, la broche repère comprenant :
- Un corps de broche ;
- Une portion distale conformée pour percer un os ; et
- Une portion proximale munie :
   ∘ D'un premier moyen de fixation pour la fixation, en utilisation, à un porte-broche d'insertion ; et
   ∘ D'un second moyen de fixation pour la fixation, en utilisation, à un porte-broche de retrait ;
   les deux moyens de fixation étant différents.

Selon des modes de réalisation particuliers :
- le premier moyen de fixation peut être un moyen de blocage radial du porte-broche d'insertion sur la broche repère, et le second moyen de fixation est un moyen de blocage axial, parallèle à la broche repère, du porte-broche de retrait sur la broche repère ;
- le premier moyen de fixation peut être de type baïonnette et le second moyen de fixation est de type filetage.
- le premier moyen de fixation de type baïonnette peut comprendre un ergot agencé radialement sur la portion proximale de la broche repère, et destiné à coulisser axialement, en utilisation, dans une rainure portée par le porte-broche d'insertion ; et le second moyen de fixation peut être un filetage agencé sur la portion proximale de la broche repère, entre le premier moyen de fixation et une extrémité proximale de la broche repère, et destiné à être vissé, en utilisation, dans un orifice taraudé porté par le porte-broche de retrait ;
- le premier moyen de fixation de type baïonnette peut comprendre une rainure agencée sur la portion proximale de la broche, et destiné à recevoir, en utilisation, un ergot agencé radialement dans une cavité portée par le porte-broche d'insertion; et le second moyen de fixation peut être un filetage agencé sur la portion proximale, entre le premier moyen de fixation et une extrémité proximale de la broche repère, et destiné à être vissé, en utilisation, dans un orifice taraudé porté par le porte-broche de retrait ; et/ou
- un méplat peut être prévu entre la partie proximale et le corps de broche.

L'invention a également pour objet un porte-broche d'insertion d'une broche repère précédente, le porte-broche d'insertion comprenant :
- Un corps cylindrique ;
- Une extrémité proximale munie d'une poignée de préhension ;
- Une extrémité distale munie :
   o d'une rainure destinée à recevoir, en utilisation, l'ergot porté par la broche repère, la rainure étant agencé pour permettre un blocage radial de l'ergot ; et
   o d'une cavité tubulaire destinée à recevoir en coulissement libre, en utilisation, le filetage porté par la broche repère.

L'invention a également pour objet un porte-broche d'insertion d'une broche repère précédent, le porte-broche d'insertion comprenant :
- Un corps cylindrique ;
- Une extrémité proximale munie d'une poignée de préhension ;
- Une extrémité distale munie :
   ∘ d'une cavité tubulaire destinée à recevoir en coulissement libre, en utilisation, le filetage porté par la broche repère ;
   ∘ d'un ergot agencé radialement dans la cavité tubulaire et destiné à s'engager, en utilisation, dans la rainure agencée sur la portion proximale de la broche repère pour permettre un blocage radial de l'ergot.

L'invention a également pour objet un porte-broche de retrait d'une broche repère précédente, le porte-broche de retrait comprenant :
- Un corps cylindrique ;
- Une extrémité proximale munie d'une poignée de préhension ;
- Une extrémité distale munie d'un orifice taraudé destiné à être vissé, en utilisation, sur le filetage porté par la broche repère.

L'invention a également pour objet un système d'insertion et de retrait précédent, le système comprenant :
- une broche repère précédente ;
- un porte-broche d'insertion précédent ; et
- un porte-broche de retrait précédent.

D'autres caractéristiques de l'invention seront énoncées dans la description détaillée ci-après, faite en référence aux dessins annexés, qui représentent, respectivement :
- la figure 1, une vue schématique en perspective d'un système d'insertion et de retrait d'une broche dite « repère » selon l'invention comprenant, en figure la un premier mode de réalisation d'une broche repère selon l'invention, en figure 1b un premier mode de réalisation d'un porte-broche d'insertion selon l'invention, en figure 1c un porte-broche de retrait selon l'invention, et en figure 1d une canule de guidage ;
- la figure 2, une vue schématique en perspective d'un deuxième mode de réalisation d'une broche repère selon l'invention ;
- la figure 3, une vue schématique en perspective d'un deuxième mode de réalisation d'un porte-broche d'insertion selon l'invention ;
- les figures 4 à 8, des vues schématiques en coupe du procédé de mise en œuvre du système selon l'invention pour l'insertion d'une broche repère selon l'invention avant opération ;
- les figures 9 à 14, des vues schématiques en coupe du procédé de mise en œuvre du système selon l'invention pour le retrait d'une broche repère selon l'invention pendant l'opération et le positionnement d'une vis pédiculaire ;
- la figure 15, une vue schématique en plan d'une vis pédiculaire canulée ; et
- la figure 16, une vue schématique en plan de la vis pédiculaire de la figure 15 munie d'un tube prolongateur.

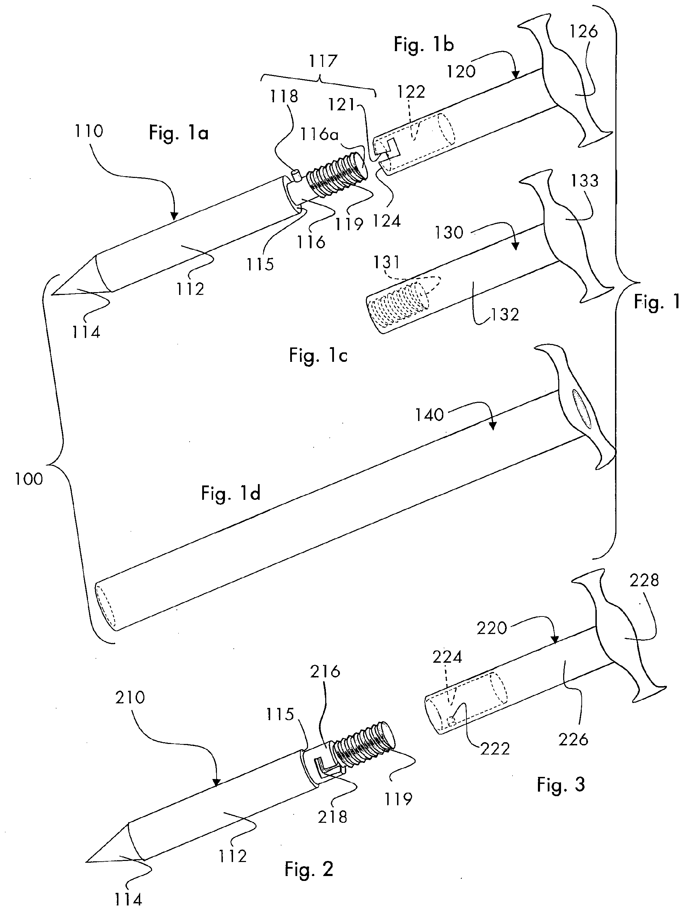

La figure 1 illustre un système 100 d'insertion et de retrait d'une broche dite « repère » 110 pour le positionnement d'une vis pédiculaire dans un os.

Le système 100 comprend une broche repère 110 (figure 1a), un porte-broche d'insertion 120 (figure 1b), un porte broche de retrait 130 (figure 1c) et une canule de guidage 140 (figure 1d).

Selon l'invention, la broche repère 110 comprend :
∘ Un corps de broche 112 ;
o Une portion distale 114 conformée pour percer un os (par exemple une pointe trocart) ; et
∘ Une portion proximale 116 munie :
   ▪ D'un premier moyen de fixation 118 pour la fixation, en utilisation, au porte-broche d'insertion 120; et
   ▪ D'un second moyen de fixation 119 pour la fixation, en utilisation, au porte-broche de retrait 130.

Selon l'invention, les deux moyens de fixation 118 et 119 sont différents, le porte-broche d'insertion 120 est muni d'un moyen de fixation 121 complémentaire du premier moyen de fixation 118 de la broche repère 110, et le porte-broche de retrait 130 est muni d'un moyen de fixation 131 complémentaire du second moyen de fixation 119 de la broche repère 110.

Le premier moyen de fixation 118 de la broche repère et du moyen de fixation 121 du porte-broche d'insertion permet, essentiellement, le couplage en rotation du porte broche d'insertion 120 sur la broche repère lors de l'insertion de ladite broche repère dans l'os. Cette insertion peut se faire par percussion, grâce à un marteau, mais également par vissage de la broche repère dans l'os. Dans ce dernier cas, le couplage en rotation du porte-broche 120 sur la broche repère 110 permet de transmettre un couple entre le porte-broche d'insertion 120 et la broche repère 110.

Le premier moyen de fixation 118 de la broche repère et du moyen de fixation 121 du porte-broche d'insertion permet également le désassemblage du porte-broche d'insertion 120 et de la broche repère 110 lorsque cette dernière est en position dans l'os.

Le second moyen de fixation 119 de la broche repère et du moyen de fixation 131 du porte-broche d'insertion permet, essentiellement, le couplage en translation du porte broche de retrait 130 sur la broche repère 110 lors du retrait de ladite broche repère 110 hors de l'os. Ce retrait ne doit pas endommager l'os, et surtout le trou réalisé lors de l'insertion. Le blocage en translation doit donc être ferme et précis. La traction nécessaire au retrait de la broche repère pouvant être forte, le second moyen de fixation doit être résistant et ne doit pas risquer de s'abîmer lors du retrait.

Ainsi, le premier moyen de fixation 118 est un moyen de blocage radial du porte-broche d'insertion 120 sur la broche repère 110, et le second moyen de fixation 119 est un moyen de blocage axial (c'est-à-dire parallèle à la direction longitudinale du corps longiligne 112 de la broche repère 110) du porte-broche de retrait 130 sur la broche repère 110.

Avantageusement, le premier moyen de fixation 118 est de type baïonnette et le second moyen de fixation 119 est de type filetage.

Dans le mode de réalisation illustré sur la figure 1a, le premier moyen de fixation 118 comprend un ergot agencé radialement sur la portion proximale 116 de la broche repère, et le moyen de fixation 121 du porte-broche d'insertion comprend une rainure située dans l'extrémité distale du support de broche d'insertion 120. Le premier moyen de fixation 118 de la broche repère est destiné à coulisser axialement, en utilisation, dans une rainure du porte-broche d'insertion .

Le second moyen de fixation 119 est un filetage agencé sur la portion proximale 116, entre le premier moyen de fixation 118 (ergot) et une extrémité proximale 116a de la broche repère 110.

Le filetage 119 est destiné à :
- être inséré en coulissement libre dans une cavité tubulaire 122 du porte-broche d'insertion lors de l'insertion de la broche repère dans l'os ;
- être vissé, pour le retrait de la broche repère, dans un moyen de fixation 131 (orifice taraudé) porté par le porte-broche de retrait 130.

Ainsi, lors de l'insertion, le filetage 119 est protégé des coups de marteau reçus par le porte-broche d'insertion 120. Lors du retrait, il est vissé dans l'orifice taraudé 131 pour solidariser fermement et précisément le porte-broche de retrait 130 sur la broche repère 110. Les nombreux points de contacts entre le filetage et le taraudage assurent un positionnement précis et une répartition large des forces de tractions, diminuant ainsi les risque d'altération du deuxième moyen de fixation lors du retrait.

Le premier moyen de fixation 118 permet l'insertion de la broche repère par « vissage » dans l'os. Pour protéger le premier moyen de fixation 118 en cas d'insertion par percussion, un méplat 115 est prévu entre la partie proximale 116 et le corps de broche 112. Ce méplat 115 est agencé pour être en contact avec le bord d'extrémité distale 124 du porte-broche d'insertion 120.

Les dimensions de la rainure 121 et de la cavité 122 sont choisies pour que l'ergot 118 de soit pas en contact avec les bords de la rainure 121 et que l'extrémité proximale de la broche repère ne soit pas en contact avec le fond de la cavité 122 lorsque le bord d'extrémité distale 124 du porte-broche d'insertion 120 est en contact avec le méplat 115. Ainsi, c'est bien ce dernier qui subit tous les chocs de percussion du marteau.

Le corps cylindrique du porte-broche d'insertion 120 comprend également, à son extrémité proximale, une poignée de préhension 126.

Pour le retrait de la broche repère, le chirurgien utilise le porte-broche de retrait 130 qui comprend :
- Un corps cylindrique 132 ;
- Une extrémité proximale munie d'une poignée de préhension 133 ; et
- Une extrémité distale munie d'un moyen de fixation 131 (orifice taraudé) destiné à être vissé, lors du retrait, sur le filetage 119 porté par la broche repère 110.

Ainsi, le chirurgien visse le porte-broche de retrait jusqu'à ce que soit l'extrémité proximale de la broche repère entre en contact avec le fond de l'orifice taraudé 131 soit que le bord de l'extrémité distale du porte-broche de retrait 130 entre en contact avec l'ergot 118 de la broche repère.

Il peut alors tirer sur la poignée de préhension 133 pour retirer la broche repère hors de l'os.

Bien entendu, pour guider à la fois l'insertion et le retrait de la broche repère, le système selon l'invention comprend une canule de guidage 140 destinée à recevoir en coulissement libre la broche repère 110, le porte-broche d'insertion 120 et le porte-broche de retrait 130.

À cette fin, la canule de guidage 140 comprend un canal intérieur 141 traversant la canule 140 de part en part. cette dernière comprend également une poignée de préhension 142, avantageusement de forme complémentaire à la poignée 126-133 des portes-broche 120-130 pour permettre un verrouillage sur la canule.

Les figures 2 et 3 illustrent un deuxième mode de réalisation d'une broche-repère 210 et d'un porte-broche d'insertion 220 selon l'invention.

Dans ce deuxième mode de réalisation, le premier moyen de fixation 218 de type baïonnette comprend une rainure agencée sur la portion proximale 216 de la broche repère 210, et le moyen de fixation 22 du porte-broche d'insertion comprenant l'ergot est agencé radialement dans une cavité 224 portée par le porte-broche d'insertion 220.

Le porte-broche d'insertion 220 de ce second mode de réalisation de broche repère comprend ainsi :
- Un corps cylindrique 226 ;
- Une extrémité proximale munie d'une poignée de préhension 228 ;
- Une extrémité distale munie :
   ∘ d'une cavité tubulaire 224 destinée à recevoir en coulissement libre, en utilisation, le filetage 119 porté par la broche repère 210 ;
   o d'un ergot 222 agencé radialement dans la cavité tubulaire 224 et destine à s'engager, en utilisation, dans la rainure 218 agencée sur la portion proximale 216 de la broche repère 210 pour permettre un blocage radial de l'ergot 222.

Le second moyen de fixation est toujours un filetage agencé sur la portion proximale de la broche repère, entre la rainure (le premier moyen de fixation) et l'extrémité proximale de la broche repère. Le porte-broche de retrait pour retirer la broche repère selon ce second mode de réalisation est similaire à celui illustré en figure 1c.

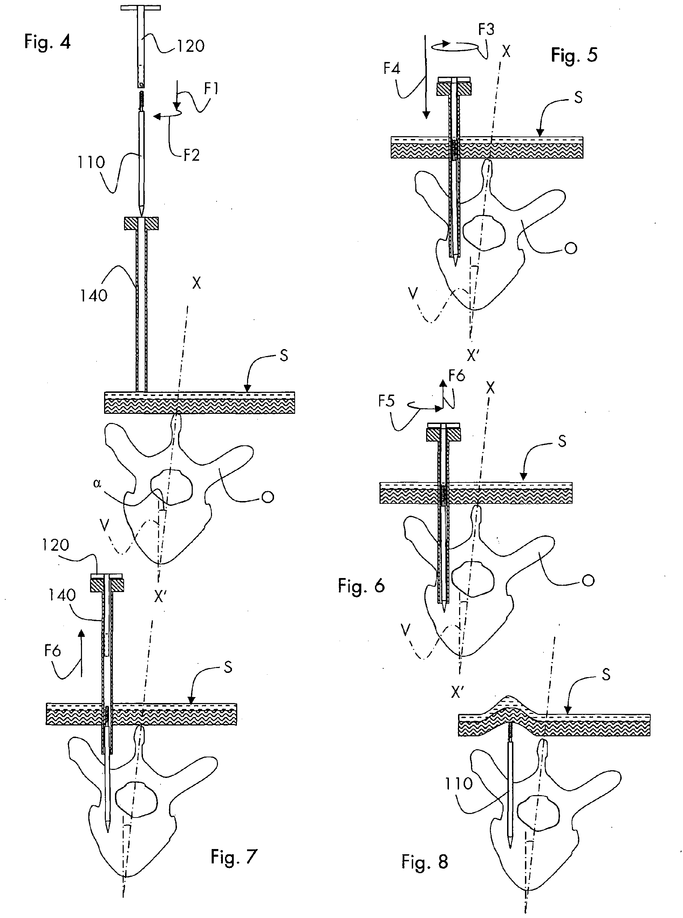

Les figures 4 à 14 illustrent le procédé de mise en œuvre permis par le système de broches repères selon l'invention.

Avant tout, le chirurgien réalise un plan d'implantation des vis pédiculaires, d'après les scans du rachis dans le plan coronal et le plan sagittal, pour être remis à l'équipe de radiologie.

Parce qu'il réduit quasi totalement le risque d'atteinte médullaire, le système selon l'invention permet de placer des implants là où c'était impossible auparavant (par exemple au sommet de la concavité). Le chirurgien peut donc définir les emplacements des vis de manière à optimiser la correction. La détermination de ces emplacements obéit à des règles anatomiques et biomécaniques précises, même si des ajustements peuvent être nécessaires pour prendre en compte les singularités d'un patient.

La première étape consiste à repérer les « vertèbres clés » du montage : vertèbre apicale et vertèbres les plus inclinées.

Puis le chirurgien détermine les vertèbres aux extrémités du montage : ces vertèbres doivent autant que possible se situer à deux ou trois étages au-dessus ou au-dessous des vertèbres les plus inclinées. Le choix de la vertèbre inférieure du montage se fait en laissant, si possible, au moins deux ou trois disques libres sous le montage.

Les deux vertèbres extrêmes doivent se trouver sur une même verticale en vue frontale, axe vers lequel va tendre la correction.

La courbure dorsale de profil doit impérativement être prise en compte : le montage ne doit pas s'arrêter au sommet d'une courbure sagittale.

Enfin, le montage ne doit pas s'arrêter à la jonction thoraco-lombaire, zone de contrainte maximale entre la zone fixée et la zone restée mobile, mais il doit au contraire la verrouiller en incluant au minimum les vertèbres L1 ou L2.

Le chirurgien réalise alors un plan d'implantation à destination du radiologue qui va positionner les broches repères 110.

Les figures 4 à 8 illustrent l'insertion de la broche repère 110.

Dans l'exemple de réalisation, la vertèbre O présente une torsion d'angle a entre son axe X-X' et la verticale V, cette torsion devant être réduite jusqu'à ce que l'angle a soit sensiblement égal à 0°.

Le patient sous anesthésie générale est sur la table du scanner qui peut être pilotée par un joystick. En suivant les indications portées par le chirurgien sur le plan d'implantation, le radiologue repère sur un scan complet du rachis les pédicules à instrumenter et il effectue le relevé des coupes correspondantes afin de localiser directement le positionnement de contrôle du scanner pendant la procédure. Il trace sur l'image l'axe du pédicule et vérifie la faisabilité de la mise en place des implants.

La précision du repérage des pédicules au scanner qui permet de réduire considérablement le risque opératoire.

La mise en place de broches repères au scanner impose un certain nombre de conditions indispensables au bon fonctionnement de la procédure. La salle de scanner devient une salle interventionnelle comme lors de toute procédure opératoire sous scanner. Le nettoyage préalable de type salle d'opération, l'asepsie du champ opératoire, la stérilisation du matériel, et la préparation de l'équipe de radiologie en procédure opératoire sont indispensables. L'anesthésie de toute l'intervention chirurgicale commence ici. L'équipement, fluides, respirateur, chariot d'anesthésie ainsi que la présence d'un anesthésiste sont indispensables au cours de cette procédure.

Pour le positionnement de la broche repère 110-210, de la même manière qu'il effectue sa visée pédiculaire lorsqu'il effectue une cimentoplastie par exemple, le radiologue interventionnel positionne la canule 140 sur la peau S du patient (figure 4). Puis il introduit dans la canule 140 la broche repère 110-210 fixée au port-broche d'insertion 120 par le premier moyen de fixation (flèches F1-F2).

Le radiologue interventionnel perce alors la peau S du patient grâce à la pointe trocart 114 de la broche repère 110-210 et introduit l'ensemble jusqu'à la surface de la vertèbre O.

Par pression-rotation (flèches F3-F4 ; figure 5) à la main ou au marteau, le radiologue interventionnel insère la broche repère dans l'axe du pédicule sur 10 mm environ pour assurer le passage de la surface externe, dure, formée d'os compact, nommé la corticale.

Alternativement, pour le perçage de la peau et de la corticale, le radiologue interventionnel peut utiliser, au lieu de l'ensemble broche repère/porte broche d'insertion, une tige trocart simple type Jamshidi avec la canule 140 type Jamshidi. Dans ce cas, il doit alors retirer la tige trocart en laissant en place la canule 140 qui l'entoure et qui sert de guide de visée pour la mise en place de l'ensemble broche repère/porte broche d'insertion.

La broche-repère 110-210, fixée sur le porte-broche 120-220 par le premier moyen de fixation 118 et glissée dans la canule 140, est ensuite enfoncée au marteau ou à l'aide d'un micromoteur dans l'axe des pédicules de chaque vertèbre O à instrumenter (et autant que possible parallèlement au plateau vertébral), selon le repérage effectué et sous contrôle scanner en temps réel (flèches F3-F4 ; figure 5).

La profondeur (P environ 50 à 60 mm) de pénétration de la broche-repère 110-210 est mesurée pour déterminer la longueur de la vis pédiculaire à placer (L = P - 1 cm).

Une fois la broche repère en place, on désactive le premier moyen de fixation 118 pour retirer le porte-broche d'insertion 120 et la canule 140.

Dans le mode de réalisation illustré, cela se fait en effectuant un mouvement de rotation/translation sur la poignée (flèches F5-F6) si la rainure 121 est en L, ou par un simple mouvement de translation si la rainure est droite et ne sert qu'à bloquer la rotation entre le porte-broche d'insertion 120 et la broche repère 110.

La longueur des broches repère 110-210 mises en place est choisie afin que l'extrémité filetée affleure sous la peau et soit palpable pour une localisation aisée (figure 8).

Lorsque toutes les broches-repères sont mises en place, le patient est adressé en salle d'opération accompagné d'un cahier de transmission, renseigné par le radiologue et remis au chirurgien. Ce cahier comporte le compte-rendu de l'intervention, avec le signalement d'éventuelles anomalies, ainsi qu'un ensemble d'images scanner présentant toutes les vertèbres instrumentées en vue axiale, plus une vue globale de face et profil. Ceci permet au chirurgien de comparer la réalité de l'implantation des broches-repères avec son plan d'intervention et de s'assurer de la faisabilité des gestes opératoires.

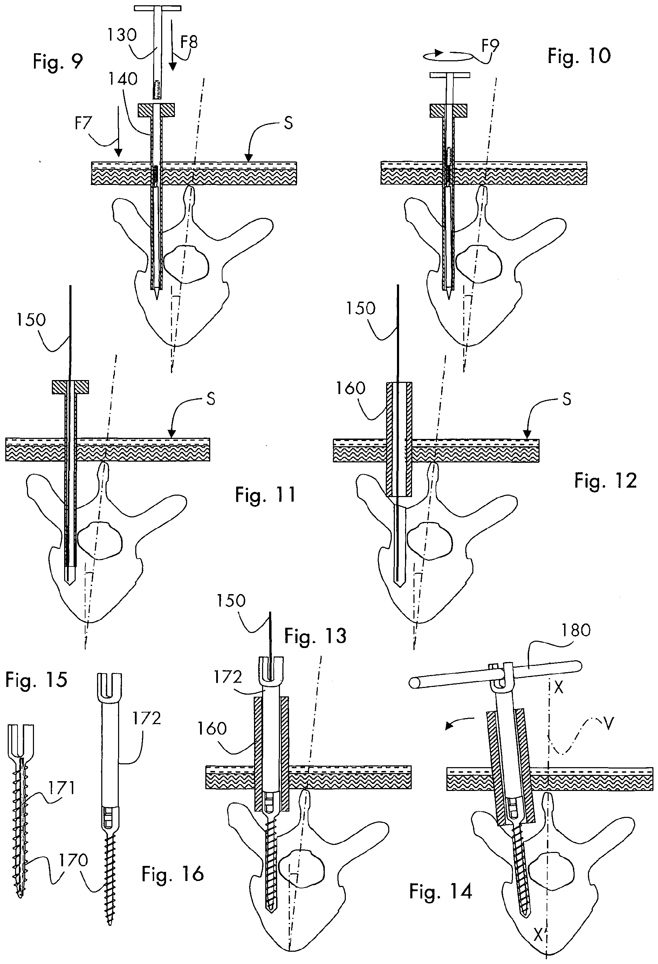

Les figures 9 à 14 illustrent la phase opératoire de pose des vis pédiculaires et des tiges de correction.

En installant le patient sur la table d'opération, on s'assure qu'aucune contrainte parasite ne viendra fausser la correction.

Le patient est installé sur la table d'opération en décubitus ventral, sans contrainte extérieure : en particulier, pas de traction. L'objectif est d'opérer la correction à partir d'une position « naturelle » ou relâchée et surtout pas précontrainte.

Le patient est accompagné de son « cahier de transmission », ensemble des coupes scanner et vues d'ensemble pour l'étape de contrôle du positionnement. Ce cahier tient lieu de documentation pour le dossier.

L'intervention est mini-invasive parce qu'on peut limiter les incisions au minimum nécessaire pour dégager la tête des broches-repères 110.

Le premier geste chirurgical est la récupération des broches repères 110 en conservant bien entendu le « trajet balistique pédiculaire » pour la mise en place des vis pédiculaires canulées.

Le point d'insertion des broches-repères 110 est repéré visuellement et à la palpation.

Pour chaque broche-repère, le chirurgien pratique une incision de 16 à 18 mm pour en dégager la tête. L'incision s'effectue en regard de la saillie sous cutanée de la broche dont on perçoit également sur la peau l'orifice de pénétration initial (voir figure 8). L'incision peut être unique ou bien allongée pour deux broches repère 110 contiguës. L'extrémité superficielle de la broche repère 110 ayant été ainsi identifiée, la canule 140 est remise en place (flèche F7 ; figure 9) en recouvrant la broche-repère dont elle suit le trajet jusque dans le pédicule de la vertèbre O.

Un porte-broche de retrait 130 est alors introduit au sein de la canule 140 (flèche F8 ; figure 9) et vissé sur l'embout fileté 119 de la broche-repère 110 (flèche F9 ; figure 10). Ainsi sécurisée, la broche-repère 110 est retirée au travers de la lumière de canule 140 qui est laissée en place.

À la place de la broche-repère, on glisse une longue tige-guide souple 150 (broche de Kirschner) dans la canule 140 (figure 11) qui est ensuite retirée avec précaution.

Les broches-repères 110 sont remplacées par des tiges-guides qui assurent une parfaite visée pédiculaire. Un guide d'introduction des vis pédiculaires est alors en place sur le trajet établi dans le pédicule par les broche repère 110 installées lors du repérage effectué en radiologie sous scanner.

Pour maintenir les tissus écartés et permettre le passage des instruments vers le pédicule, on place un premier tube dilatateur 160 autour de la tige-guide 150 jusqu'au contact du pédicule (figure 12).

En pratique, le tube dilatateur est constitué de plusieurs tubes coaxiaux de diamètres croissants. Ainsi, un premier tube de petit diamètre est introduit autour de la tige-guide, puis un deuxième tube de diamètre supérieur, autour du premier, puis un troisième tube de diamètre encore supérieur autour du deuxième.

Le chirurgien prend alors une vis pédiculaire 170 (figure 15) avantageusement canulée, c'est-à-dire qu'elle présente un canal 171 central ou latéral de diamètre suffisant pour recevoir la tige-guide 150.

Lors de l'insertion et de la réduction de la torsion, la vis pédiculaire 170 est fixée au bout d'un prolongateur tubulaire 172 (figure 16).

Afin de faciliter le passage de la vis pédiculaire 170 au travers de la corticale, on peut utiliser une pointe carrée canulée guidée par le tube dilatateur.

Dans l'axe de la tige-guide 150 et donc du pédicule, on peut avantageusement forer au taraud un avant-trou pour faciliter le passage de la vis 170.

Fixée au bout d'un prolongateur tubulaire 172, la vis pédiculaire 170 est guidée par la tige-guide 150 jusqu'à l'avant-trou dans lequel elle est vissée jusqu'à la profondeur souhaitée. La tige-guide 150 peut être retirée dès que la vis est bien engagée dans le pédicule.

Les tubes prolongateurs peuvent être laissés en place pour servir de poignées qui permettront d'ajuster la position des vertèbres, comme schématisé à la figure 14.

Le chirurgien réduit la torsion de la vertèbre soit à la main, soit à l'aide d'un appareil dédié, jusqu'à ce que l'axe X-X' de la vertèbre soit vertical, dans le cas illustré.

La vertèbre est ainsi maintenue en place grâce à une tige de correction 180.

Plus en détail, cette opération de redressement s'effectue en plusieurs étapes.

Tout d'abord, on réalise un horizontalisation des vertèbres les plus inclinées grâce à un appareil de correction d'angle (par exemple le Marnay's Angle Corrector) : ce type d'appareil, utilisé dans le cas de courbure extrêmes difficiles à réduire, permet, grâce une double crémaillère du côté concave et du côté convexe, de corriger la courbure globale entre les deux vertèbres les plus inclinées et à maintenir cette correction jusqu'à la mise en place des tiges.

Ensuite on effectue une distraction des vertèbres en cas de pincement prononcé.

L'action du distracteur, par rapport à celle de l'horizontaliseur, est locale et latérale, au niveau segmentaire. Elle permet de corriger des pincements importants entre deux vertèbres consécutives.

Une fois les corrections d'angle effectuées, on place des tiges de correction : tiges de courbure et tiges de renfort.

Les tiges de courbure en titane sont relativement souples afin de faciliter leur insertion et d'obtenir une première réduction de la scoliose sans contraintes excessives.

Les tiges de renfort en chrome-cobalt sont plus rigides que les tiges de correction dont elles viennent compléter et fixer l'effet correctif.

Toutes les tiges de correction doivent être cintrées de manière à anticiper la courbure sagittale visée (lordose dorsale) tout en épousant au mieux la courbure frontale à corriger.

Le cintrage de la tige de courbure concave facilite son insertion dans les têtes de vis, puis il donne à la colonne la courbure sagittale souhaitée tout en réduisant la courbure frontale lorsque, une fois mise en place, la tige est pivotée de 90°, de sorte que sa courbure passe du plan frontal au plan sagittal.

La relative flexibilité de la tige en titane permet d'amplifier sa courbure, en même temps que l'on amorce la réduction de la courbure vertébrale, pour l'amener à la rencontre des têtes des vis pédiculaires.

La mise en place des tiges s'effectue de la manière suivante.

La première tige à placer est la tige de courbure du côté de la concavité. Avant d'être insérée, cette tige doit être cintrée de manière à anticiper la courbure sagittale visée (cyphose et lordose dorsales) tout en épousant au mieux la courbure frontale à corriger ; elle est également coupée à la bonne longueur.

Tenue par un préhenseur, la tige de courbure concave est introduite par abord percutané, du haut vers le bas (sens crânio-caudal), en jouant sur les tubes prolongateurs pour mettre la tulipe de la tête de la vis en face de l'extrémité de la tige.

Il est parfois nécessaire d'élargir une incision pour insérer la tige.

Notons qu'il pourrait être préférable de procéder à l'insertion de la tige du bas vers le haut, c'est-à-dire en partant du bassin qui est la référence naturelle. Mais la lordose lombaire et les fesses sont une gêne pour présenter la tige, alors que la tête, moins large et inclinée vers l'avant, l'est beaucoup moins.

Lorsque la tige est insérée dans la tête de la vis pédiculaire, on met en place l'écrou de verrouillage, mais on ne verrouille pas : il est en effet essentiel de conserver jusqu'au bout de l'intervention un maximum de liberté dans l'ajustement de la position des vertèbres afin d'optimiser la correction.

La tige de courbure convexe est ensuite introduite juste après la tige de courbure concave ; elle va servir de contre-appui facilitant les manœuvres de « dérotation » et d'horizontalisation des vertèbres.

Une fois que les deux tiges de correction ont été mises en place, la tige de courbure concave est pivotée de 90° autour de son axe principal, ce qui, conjointement à une action sur les tubes prolongateurs, force la dérotation et l'horizontalisation des vertèbres.

Cette rotation est contrôlée par le préhenseur de tige dont le plan de fixation doit coïncider avec le plan de cintrage, cintrage qui *in fine* détermine la courbure antéro-postérieure de la colonne. Ainsi, lorsque le préhenseur sera perpendiculaire au dos du patient, on saura que la rotation effectuée est bien de 90°.

Lorsque la tige de courbure concave a été pivotée de 90°, on peut la fixer en serrant l'écrou de verrouillage dans la tête de la vis pédiculaire de la vertèbre supérieure du montage. Aucune autre tête de vis ne sera verrouillée à ce stade afin de permettre à la tige de coulisser sans nouvelles contraintes jusqu'à l'achèvement de la réduction de la courbure.

Après que la tige de courbure concave a été pivotée de 90°, la tige de courbure convexe est pivotée de 90° à son tour et de la même manière, ce qui parfait la correction.

Lorsque les tiges sont engagées dans les vis, deux cas de figure peuvent se produire : la force de rappel élastique des tiges l'emporte sur celle de la colonne, ou le contraire.

Si la force de rappel de la tige est supérieure à celle de la colonne, en retrouvant sa forme initiale, la tige réduit encore la courbure vertébrale. Puis la rotation à 90° de la tige poursuit la réduction de la courbure qui est achevée par la mise en place de la tige convexe.

Si la force de rappel de la tige est inférieure à celle de la colonne, la tige ne reprend que partiellement la courbure de la colonne. Et même après la rotation de 90°, la tige reste courbée dans le plan frontal. Dans ces conditions, la tige convexe ne parvient pas non plus à réduire la courbure de la colonne. Les tiges de renfort sont alors nécessaires.

Dans ce cas, du côté de la concavité on renforce la correction de la courbure ; du côté de la convexité on renforce la correction de la torsion manifestée par la gibbosité.

Chacune de ces deux tiges est montée sur trois ou quatre connecteurs latéraux fixés sur les tiges de correction. La première tige de renfort est placée du côté de la concavité pour fixer la réduction préliminaire assurée par la tige de correction. La deuxième tige de renfort, placée du côté de la convexité, sert à maintenir un couple de rotation qui corrige la gibbosité.

Les tiges mises en place ayant encore une certaine liberté de mouvement, on peut ajuster la correction avant de serrer progressivement les écrous.

Tout doit pouvoir bouger le plus longtemps possible, pour que l'on puisse ajuster et optimiser la réduction de la scoliose. Quand tout est en place, alors seulement on peut verrouiller le montage en serrant les écrous des vis pédiculaires et des connecteurs.

Une greffe osseuse était absolument indispensable en technique traditionnelle (comme la mise en place d'une tige de Harrington) pour que la fusion vertébrale compense les efforts considérables exercés sur le montage.

Grâce au système selon l'invention, il est possible d'équiper des vertèbres qu'on n'équipait pas jusqu'à présent, ce qui permet une meilleure réduction de la courbure, une optimisation de la répartition des charges sur quatre tiges et la préservation du haubanage musculaire.

Tout ceci rend la greffe moins indispensable, mais elle permet de sécuriser les ancrages et accélérer la fusion des vertèbres, en particulier au niveau des « zones charnières », c'est-à-dire des zones de changement de contraintes.

Tout le long des tiges de fusion, après avoir avivé les transverses, on injecte donc des greffons : os de banque ou substitut osseux (hydroxyapatite), plus moelle osseuse.

Grâce au système selon l'invention, la précision de l'implantation des broches repère, réduit quasiment totalement le risque de fausse route et d'intrusion dans le canal médullaire.

Le temps d'ouverture et la surface d'exposition étant extrêmement réduits par rapport aux techniques traditionnelles, le risque infectieux l'est tout autant.

Le caractère mini-invasif de l'intervention élimine pratiquement tout risque hémorragique.

Enfin, l'atteinte des muscles dorsaux est minime et n'altère pas leur capacité à soutenir la colonne vertébrale; elle n'entraîne aucun risque de nécrose musculaire.

Le système selon l'invention permet de scinder l'opération en deux : une phase en salle de radiologie et une phase en salle d'opération.

Cela permet de limiter l'exposition du personnel soignant au rayonnement. En effet, dans la procédure classique, tout le personnel (quatre à cinq personnes) du bloc opératoire est exposé au rayonnement, alors qu'avec la procédure permise par le système selon l'invention, seules les personnes présentes en salle de radiologie (deux personnes) sont exposées.

Sur le plan économique, la procédure permise par le système selon l'invention est très avantageuse car elle utilise un scanner en salle de radiologie comme il en existe dans tous les établissements hospitaliers, scanner dont l'usage ne lui est pas réservé et qui peut donc fonctionner en permanence pour des indications très diverses.

Ceci est à comparer avec l'utilisation d'un scanner per-opératoire comme le O-ARM dont l'usage est sensiblement plus restreint et dont le prix actuel est d'environ 1 million d'euros, auquel il faut ajouter celui d'une table en carbone d'environ 400 000 euros, ainsi que celui d'un éventuel robot d'environ 1 million d'euros, ces matériels restant immobilisés plusieurs heures au cours d'une procédure sans pouvoir être utilisé pour un autre patient avant la fin de l'opération.

D'autre part, si la durée de l'intervention « hors tout » (radiologie + chirurgie) est à peu près équivalente à celle d'une technique traditionnelle, le temps passé au bloc opératoire est réduit de moitié. Considérant qu'il faut deux personnes en radiologie et quatre ou cinq au bloc, sur 6 heures d'intervention au total, l'économie est de 6 à 9 h de temps de personnels.

Enfin, l'intervention de positionnement des vis pédiculaires et du redressement de la colonne vertébrale nécessite du personnel moins qualifié puisque les trous sont déjà fait par le radiologue.

Faiblement traumatisante, l'intervention avec un système selon l'invention est suivie d'une convalescence rapide nécessitant une moindre prise en charge post-opératoire.

## Revendications

1. Système d'insertion et de retrait (100) d'une broche repère (110, 210) pour le positionnement d'une vis pédiculaire (170) dans un os (O), **caractérisé en ce qu'**il comprend :
• Une broche repère (110, 210) comprenant:
∘ Un corps de broche (112) ;
∘ Une portion distale (114) conformée pour percer un os ; et
∘ Une portion proximale (116) munie :
▪ D'un premier moyen de fixation (118) pour la fixation, en utilisation, à un porte-broche d'insertion (120) ; et
▪ D'un second moyen de fixation (119) pour la fixation, en utilisation, à un porte-broche de retrait (130) ;
les deux moyens de fixation (118, 119) étant différents ;
• un porte-broche d'insertion (120) muni d'un moyen de fixation (121) complémentaire du premier moyen de fixation (117) de la broche repère ;
• un porte-broche de retrait muni d'un moyen de fixation (131) complémentaire du second moyen de fixation (119) de la broche repère ; et
• une canule de guidage (140) destinée à recevoir en coulissement libre la broche repère (110), le porte-broche d'insertion (120) et le porte-broche de retrait (130).

2. Broche repère (110, 210) pour le positionnement d'une vis pédiculaire dans un os, **caractérisé en ce qu'**elle comprend :
• Un corps de broche (112) ;
• Une portion distale (114) conformée pour percer un os ; et
• Une portion proximale (116) munie :
∘ D'un premier moyen de fixation (118) pour la fixation, en utilisation, à un porte-broche d'insertion (120) ; et
∘ D'un second moyen de fixation (119) pour la fixation, en utilisation, à un porte-broche de retrait (130) ;
les deux moyens de fixation (118, 119) étant différents.

3. Broche repère (110, 210) selon la revendication 2, dans laquelle le premier moyen de fixation (118) est un moyen de blocage radial (118, 121) du porte-broche d'insertion (120) sur la broche repère (110), et le second moyen de fixation (119) est un moyen de blocage axial, parallèle à la broche repère, du porte-broche de retrait (130) sur la broche repère (110).

4. Broche repère (110, 210) selon la revendication 3, dans laquelle le premier moyen de fixation est de type baïonnette et le second moyen de fixation est de type filetage.

5. Broche repère (110) selon la revendication 4, dans laquelle:
• le premier moyen de fixation de type baïonnette comprend un ergot (118) agencé radialement sur la portion proximale (116) de la broche repère, et destiné à coulisser axialement, en utilisation, dans une rainure (121) portée par le porte-broche d'insertion (120) ; et
• le second moyen de fixation est un filetage (119) agencé sur la portion proximale (116) de la broche repère, entre le premier moyen de fixation (118) et une extrémité proximale de la broche repère, et destiné à être vissé, en utilisation, dans un orifice taraudé (131) porté par le porte-broche de retrait (130).

6. Broche repère (210) selon la revendication 4, dans laquelle:
• le premier moyen de fixation de type baïonnette comprend une rainure (218) agencée sur la portion proximale (216) de la broche, et destiné à recevoir, en utilisation, un ergot (222) agencé radialement dans une cavité (224) portée par le porte-broche d'insertion (220) ; et
• le second moyen de fixation est un filetage (119) agencé sur la portion proximale (216), entre le premier moyen de fixation (218) et une extrémité proximale de la broche repère, et destiné à être vissé, en utilisation, dans un orifice taraudé (131) porté par le porte-broche de retrait (130).

7. Broche repère (110, 210) selon l'une quelconque des revendications 2 à 6, dans laquelle un méplat (115) est prévu entre la partie proximale et le corps de broche.

8. Porte-broche d'insertion (120) d'une broche repère selon la revendication 5, **caractérisé en ce qu'**il comprend :
• Un corps cylindrique ;
• Une extrémité proximale munie d'une poignée de préhension (126) ;
• Une extrémité distale (124) munie :
o d'une rainure (121) destinée à recevoir, en utilisation, l'ergot (118) porté par la broche repère (110), la rainure (121) étant agencé pour permettre un blocage radial de l'ergot (118) ; et
o d'une cavité tubulaire (122) destinée à recevoir en coulissement libre, en utilisation, le filetage (119) porté par la broche repère.

9. Porte-broche d'insertion (220) d'une broche repère (210) selon la revendication 6, **caractérisé en ce qu'**il comprend :
• Un corps cylindrique (226) ;
• Une extrémité proximale munie d'une poignée de préhension (228) ;
• Une extrémité distale munie :
∘ d'une cavité tubulaire (224) destinée à recevoir en coulissement libre, en utilisation, le filetage (119) porté par la broche repère (210) ;
o d'un ergot (222) agencé radialement dans la cavité tubulaire (224) et destiné à s'engager, en utilisation, dans la rainure (218) agencée sur la portion proximale (216) de la broche repère (210) pour permettre un blocage radial de l'ergot (222).

10. Porte-broche de retrait d'une broche repère (110, 210) selon la revendication 5 ou 6, **caractérisé en ce qu'**il comprend :
• Un corps cylindrique (132) ;
• Une extrémité proximale munie d'une poignée de préhension (133) ;
• Une extrémité distale munie d'un orifice taraudé (131) destiné à être vissé, en utilisation, sur le filetage (119) porté par la broche repère (110, 210).

11. Système d'insertion et de retrait selon la revendication 1, **caractérisé en ce qu'**il comprend :
• une broche repère selon l'une quelconque des revendications 2 à 7;
• un porte-broche d'insertion selon l'une quelconque des revendications 8 ou 9 ; et
• un porte-broche de retrait selon la revendication 10.

## Patentansprüche

1. System zum Einsetzen und Herausziehen (100) eines Ausrichtungsstifts (110, 210) zur Positionierung einer Pedikelschraube (170) in einem Knochen (O), **dadurch gekennzeichnet, dass** es Folgendes umfasst:
• einen Ausrichtungsstift (110, 210), umfassend:
o einen Stiftkörper (112);
o einen angepassten distalen Abschnitt (114) zum Durchbohren eines Knochens; und
o einen proximalen Abschnitt (116) mit:
▪ einem ersten Befestigungsmittel (118) zum Fixieren, im Einsatz, an einem Einsetzstiftträger (120); und
▪ einem zweiten Befestigungsmittel (119) zum Fixieren, im Einsatz, an einem Ausziehstiftträger (130);
wobei die beiden Befestigungsmittel (118, 119) unterschiedlich sind;
• eine Einsetzstiftträger (120) mit einem Befestigungsmittel (121) zusätzlich zu dem ersten Befestigungsmittel (117) des Ausrichtungsstifts;
• eine Ausziehstiftträger mit einem Befestigungsmittel (131) zusätzlich zu dem zweiten Befestigungsmittel (119) des Ausrichtungsstifts; und
• eine Führungskanüle (140) zum frei verschieblichen Aufnehmen des Ausrichtungsstifts (110), des Einsetzstiftträgers (120) und des Ausziehstiftträgers (130).

2. Ausrichtungsstift (110, 210) zum Positionieren einer Pedikelschraube in einem Knochen, **dadurch gekennzeichnet, dass** er Folgendes umfasst:
• einen Stiftkörper (112);
• einen angepassten distalen Abschnitt (114) zum Durchbohren eines Knochens; und
• einen proximalen Abschnitt (116) mit:
o einem ersten Befestigungsmittel (118) zum Fixieren, im Einsatz, an einem Einsetzstiftträger (120); und
o einem zweiten Befestigungsmittel (119) zum Fixieren, im Einsatz, an einem Ausziehstiftträger (130);
wobei die beiden Befestigungsmittel (118, 119) unterschiedlich sind;

3. Ausrichtungsstift (110, 210) nach Anspruch 2, wobei das erste Befestigungsmittel (118) ein Radialverriegelungsmittel (118, 121) des Einsetzstiftträgers (120) auf dem Ausrichtungsstift (110) ist und das zweite Befestigungsmittel (119) ein Radialverriegelungsmittel, parallel zum Ausrichtungsstift, des Ausziehstiftträgers (130) auf dem Ausrichtungsstift (110) ist.

4. Ausrichtungsstift (110, 210) nach Anspruch 3, wobei das erste Befestigungsmittel als Bajonett ausgestattet ist und das zweite Befestigungsmittel als Gewinde ausgestattet ist.

5. Ausrichtungsstift (110) nach Anspruch 4, wobei:
• das erste Befestigungsmittel, das als Bajonett ausgestattet ist, einen radial auf dem proximalen Abschnitt (116) des Ausrichtungsstifts angeordneten und, im Einsatz, zum axialen Verschieben in einer Aussparung (121) an dem Einsetzstiftträger (120) bestimmten Vorsprung (118) umfasst; und
• das zweite Befestigungsmittel ein zwischen dem ersten Befestigungsmittel (118) und einem proximalen Ende des Ausrichtungsstifts auf dem proximalen Abschnitt (116) des Ausrichtungsstifts angeordnetes Gewinde (119) ist und, im Einsatz, zum Einschrauben in ein Gewindeloch (131) an dem Ausziehstiftträger (130) bestimmt ist.

6. Ausrichtungsstift (210) nach Anspruch 4, wobei:
• das erste Befestigungsmittel, das als Bajonett ausgestattet ist, eine auf dem proximalen Abschnitt (216) des Stifts angeordnete und, im Einsatz, zum Aufnehmen einen radial in einem Hohlraum (224) an dem Einsetzstiftträger (220) angeordneten Vorsprung (222) bestimmte Aussparung (218) umfasst;
und
• das zweite Befestigungsmittel ein zwischen dem ersten Befestigungsmittel (218) und einem proximalen Ende des Ausrichtungsstifts auf dem proximalen Abschnitt (216) angeordnetes Gewinde (119) ist und, im Einsatz, zum Einschrauben in ein Gewindeloch (131) an dem Ausziehstiftträger (130) bestimmt ist.

7. Ausrichtungsstift (110, 210) nach einem der Ansprüche 2 bis 6, wobei zwischen dem proximalen Teil und dem Stiftkörper eine Abflachung (115) vorgesehen ist.

8. Einsetzstiftträger (120) eines Ausrichtungsstifts nach Anspruch 5, **dadurch gekennzeichnet, dass** es Folgendes umfasst:
• einen zylindrischen Körper;
• ein proximales Ende mit einem Handgriff (126);
• ein distales Ende (124) mit:
o einer Aussparung (121), die, im Gebrauch, zum Aufnehmen des Vorsprungs (118) an dem Ausrichtungsstift (110) bestimmt ist, wobei die Aussparung (121) so angeordnet ist, dass sie eine radiale Verriegelung des Vorsprungs (118) ermöglicht; und
o einem röhrenförmigen Hohlraum (122), der, im Einsatz, zum frei verschieblichen Aufnehmen des Gewindes (119) an dem Ausrichtungsstift bestimmt ist.

9. Einsetzstiftträger (220) eines Ausrichtungsstifts (210) nach Anspruch 6, **dadurch gekennzeichnet, dass** es Folgendes umfasst:
• einen zylindrischen Körper (226);
• ein proximales Ende mit einem Handgriff (228);
• ein distales Ende mit:
o einem röhrenförmigen Hohlraum (224), der, im Einsatz, zum frei verschieblichen Aufzunehmen des Gewindes (119) an dem Ausrichtungsstift (210) bestimmt ist;
o einem Vorsprung (222), der radial in dem röhrenförmigen Hohlraum (224) angeordnet und, im Einsatz, zum Eingreifen in der Aussparung (218), die auf dem proximalen Abschnitt (216) des Ausrichtungsstifts (210) angeordnet ist, bestimmt ist, um eine radiale Verriegelung des Vorsprungs (222) zu ermöglichen.

10. Ausziehstiftträger eines Ausrichtungsstifts (110, 210) nach Anspruch 5 oder 6, **dadurch gekennzeichnet, dass** es Folgendes umfasst:
• einen zylindrischen Körper (132);
• ein proximales Ende mit einem Handgriff (133);
• ein distales Ende mit einem Gewindeloch (131), das, im Einsatz, zum Einschrauben auf das Gewinde (119) an dem Ausrichtungsstift (110, 210) bestimmt ist.

11. System zum Einsetzen und Herausziehen nach Anspruch 1, **dadurch gekennzeichnet, dass** es Folgendes umfasst:
• einen Ausrichtungsstift nach einem der vorhergehenden Ansprüche 2 bis 7;
• einen Einsetzstiftträger nach einem der vorhergehenden Ansprüche 8 oder 9; und
• einen Stiftträger zum Herausziehen nach Anspruch 10.

## Claims

1. System for inserting and removing (100) a reference pin (110, 210) for positioning a pedicle screw (170) in a bone (O), **characterized in that** said system comprises:
• a reference pin (110, 210) comprising:
o a pin body (112);
o a distal portion (114) that is shaped so as to pierce a bone; and
o a proximal portion (116) provided with:
▪ a first fixing means (118) for fixing, during use, to an insertion pin holder (120); and
▪ a second fixing means (119) for fixing, during use, to a removal pin holder (130);
the two fixing means (118, 119) being different;
• an insertion pin holder (120) provided with a fixing means (121) that is complementary to the first fixing means (117) of the reference pin;
• a removal pin holder provided with a fixing means (131) that is complementary to the second fixing means (119) of the reference pin; and
• a guide cannula (140) which is intended for receiving, in a freely sliding manner, the reference pin (110), the insertion pin holder (120) and the removal pin holder (130).

2. Reference pin (110, 210) for positioning a pedicle screw in a bone, **characterized in that** said pin comprises:
• a pin body (112);
• a distal portion (114) that is shaped so as to pierce a bone; and
• a proximal portion (116) provided with:
o a first fixing means (118) for fixing, during use, to an insertion pin holder (120); and
o a second fixing means (119) for fixing, during use, to a removal pin holder (130); the two fixing means (118, 119) being different.

3. Reference pin (110, 210) according to claim 2, wherein the first fixing means (118) is a radial blocking means (118, 121) of the insertion pin holder (12) on the reference pin (110), and the second fixing means (119) is an axial blocking means, in parallel with the reference pin, of the removal pin holder (130) on the reference pin (110).

4. Reference pin (110, 210) according to claim 3, wherein the first fixing means is of the bayonet type and the second fixing means is of the thread type.

5. Reference pin (110) according to claim 4, wherein:
• the first fixing means of the bayonet type comprises a lug (118) which is arranged radially on the proximal portion (116) of the reference pin and is intended to slide axially, during use, in a groove (121) borne by the insertion pin holder (120); and
• the second fixing means is a thread (119) arranged on the proximal portion (116) of the reference pin, between the first fixing means (118) and a proximal end of the reference pin, and intended to be screwed, during use, into a tapped opening (131) borne by the removal pin holder (130).

6. Reference pin (210) according to claim 4, wherein:
• the first fixing means of the bayonet type comprises a groove (218) arranged on the proximal portion (216) of the pin and intended to receive, during use, a lug (222) which is arranged radially in a cavity (224) borne by the insertion pin holder (220); and
• the second fixing means is a thread (119) which is arranged on the proximal portion (216), between the first fixing means (218) and a proximal end of the reference pin and is intended to be screwed, during use, into a tapped opening (131) borne by the removal pin holder (130).

7. Reference pin (110, 210) according to any of claims 2 to 6, wherein a flattened region (115) is provided between the proximal part and the pin body.

8. Insertion pin holder (120) of a reference pin according to claim 5, **characterized in that** said holder comprises:
• a cylindrical body;
• a proximal end provided with a gripping handle (126);
• a distal end (124) provided with:
o a groove (121) that is intended to receive, during use, the lug (118) borne by the reference pin (110), the groove (121) being arranged so as to allow for radial blocking of the lug (118); and
o a tubular cavity (122) that is intended to receive, in a freely sliding manner, during use, the thread (119) borne by the reference pin.

9. Insertion pin holder (220) of a reference pin (210) according to claim 6, **characterized in that** said holder comprises:
• a cylindrical body (226);
• a proximal end provided with a gripping handle (228);
• a distal end provided with:
o a tubular cavity (224) that is intended to receive, in a freely sliding manner, during use, the thread (119) borne by the reference pin (210);
o a lug (222) that is arranged radially in the tubular cavity (224) and is intended to engage, during use, in the groove (218) arranged on the proximal portion (216) of the reference pin (210) in order to allow for radial blocking of the lug (222).

10. Removal pin holder of a reference pin (110, 210) according to either claim 5 or claim 6, **characterized in that** said holder comprises:
• a cylindrical body (132);
• a proximal end provided with a gripping handle (133);
• a distal end provided with a tapped opening (131) which is intended to be screwed, during use, to the thread (119) borne by the reference pin (110, 210).

11. Insertion and removal system according to claim 1, **characterized in that** it comprises:
• a reference pin according to any of claims 2 to 7;
• an insertion pin holder according to either claim 8 or claim 9; and
• a removal pin holder according to claim 10.
